(19) Europäisches Patentamt
European Patent Office
Office européen des brevets

(11) Publication number: 0 154 173 B1

# (12) EUROPEAN PATENT SPECIFICATION

(45) Date of publication of patent specification: 08.01.92

(51) Int. Cl.[5]: C07D 475/06, C07D 475/04, C07D 475/08, C07D 475/00, A61K 31/505

(21) Application number: 85101131.2

(22) Date of filing: 04.02.85

The file contains technical information submitted after the application was filed and not included in this specification

## (54) Pteridine derivatives, an anticancer composition containing same and a process for the production of said derivatives.

(30) Priority: 03.02.84 US 576581

(43) Date of publication of application: 11.09.85 Bulletin 85/37

(45) Publication of the grant of the patent: 08.01.92 Bulletin 92/02

(84) Designated Contracting States: BE CH DE FR GB IT LI LU NL SE

(56) References cited:
US-A- 2 537 006
US-A- 3 128 273
US-A- 4 077 957
US-A- 4 374 987

CHEMICAL ABSTRACTS, vol. 101, no. 25, 17th December 1984, page 26, column 2, abstract no. 222182d, Columbus, Ohio, US; M. KHALED et al.: "2,4-Diamino-6-(bis-2-chloroethyl)aminomethyl-pteridine. A new potent anticancer drug"

(73) Proprietor: RESEARCH CORPORATION TECHNOLOGIES, INC.
6840 East Broadway Boulevard
Tucson Arizona 85710(US)

(72) Inventor: Khaled, Mohammad Abu
405 Elder Oaks Way
Birmingham Alabama(US)
Inventor: Benington, Frederick
500 Edgeland Place
Birmingham Alabama(US)
Inventor: Morin, Richard D.
1043 Ivy Creek Trail
Birmingham Alabama(US)

(74) Representative: Brauns, Hans-Adolf, Dr. rer. nat. et al
Hoffmann, Eitle & Partner, Patentanwälte Arabellastrasse 4
W-8000 München 81(DE)

EP 0 154 173 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF MEDICINAL CHEMISTRY, vol. 18, no. 9, 1975, pages 909-912, US; M. CHAYKOVSKY et al.: "Methotrexate analogs. 6. replacement of glutamic acid by various amino acid esters and amines"

Cancer Chemotherapy and Pharmacology (1984), 13: 73-74

## Description

This invention relates to the pteridines generally, and more particularly, to pteridine derivatives bearing the particular alkylating moiety in the 6-position on the pteridine nucleus as hereinafter more fully disclosed. The invention also relates to pharmaceutical preparations containing the aforesaid pteridine derivatives and to a process for preparing same.

U.S.Patent Nos. 4,077,957 and 4,374,987 describe procedures for preparing pteridine compounds including the anticancer drug methotrexate which possesses the structure

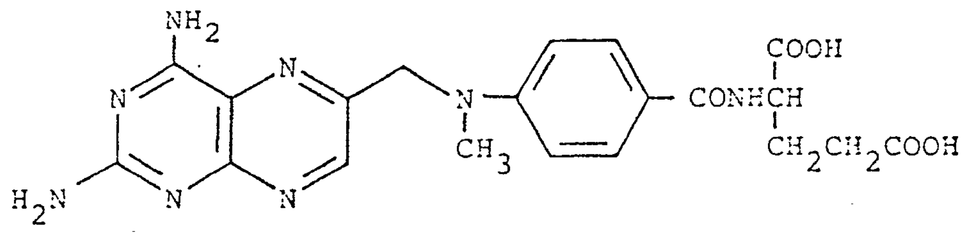

N- P-([2,4-diamino-6-pteridinyl)-methyl] methylamino)benzoyl glutamic acid and analogous compounds such as aminopteridine

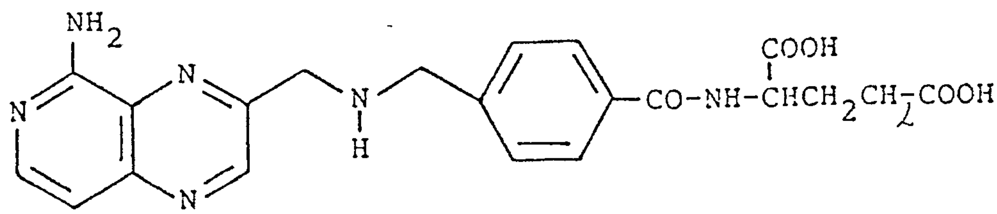

Methotrexate has in recent years become a prominent drug in the treatment of a variety of cancers (C.B. Pratt, et al., Cancer Chemother. Rep., Part 3, 6, 13 (1975).

Analogs of methotrexate are discussed in Journal of Medicinal Chemistry, 1975, vol. 18, No. 9, 909-912.

In accordance with the present invention, pteridine derivatives and their pharmaceutically acceptable salts having potent anticancer activity and which are significantly more target specific than methotrexate and its analogs are provided. The pteridine derivatives of this invention possess the structural formula

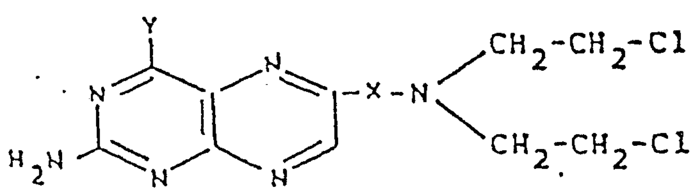

wherein
Y is $NH_2$, OH or SH, and
X is

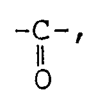

or $-CH_2-$.

The pteridine derivatives of the present invention can be readily prepared by those of ordinary skill in the art employing known and conventional syntheses. Thus, for example, preparation of the 2,4-diaminopteridine derivatives in which X is

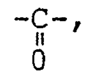

i.e. compounds of the formula

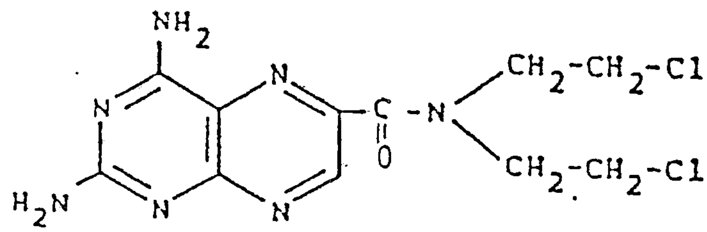

(I)

can be obtained by reacting the corresponding carboxy acid, under amide-forming conditions, with an appropriate amine, to provide the desired derivative. An example of the sequence of reactions is illustrated as follows:

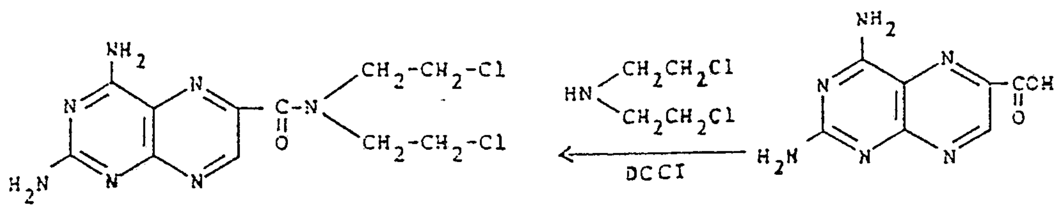


2,4-diamino-6-[(bis-2-chloroethyl)carboxamido]pteridine

The starting compound of the above scheme may be prepared by oxidation of 2,4-diamino-6-(hydroxymethyl)-pteridine, a known intermediate whose preparation is described in U.S. Patent No. 4,077,957.

The amide forming conditions referred to herein involve the use of known derivatives of the described acids, such as the acyl halides, anhydrides, mixed anhydrides, lower alkyl esters, carbodiimides, carbonyl diimidazoles, and the like. The reactions are carried out in organic solvents such as acetonitrile, tetrahydrofuran, dioxane, acetic acid, methylene chloride, ethylene chloride and similar such solvents. The abide forming reaction will occur at room temperature or at elevated temperature. The use of elevated temperature is for convenience in that it permits somewhat shortened reaction periods. Temperatures ranging from 0°C up to the reflux temperature of the reaction system can be used. As a further convenience the amide forming reaction can be effected in the presence of a base such as tertiary organic amines, e.g., trimethylamine, pyridine, picolines and the like, particularly where hydrogen halide is formed by the amide-forming reaction, e.g., acyl halide and amino compound. Of course, in those reactions where hydrogen halide is produced, any of the commonly used hydrogen halide acceptors can also be used.

The 2,4-diaminopteridine derivatives in which X is $-CH_2-$, i.e., compounds of the formula

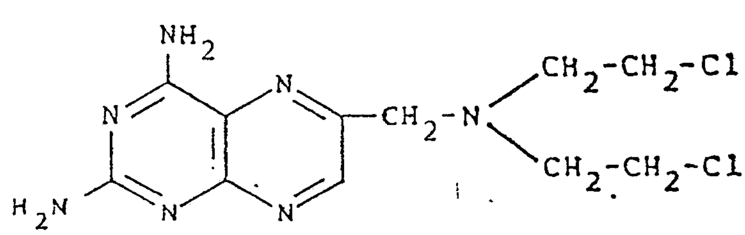

(II)

can also be readily prepared via known chemical syntheses.

For example, a 6-halomethyl derivative of 2, 4-diamino-6 (hydroxymethyl)- pteridine may be reacted with an appropriate amine, e.g., bis(2-chloroethyl) amine, to provide the desired derivative.
An exemplary sequence of reactions is illustrated as follows:

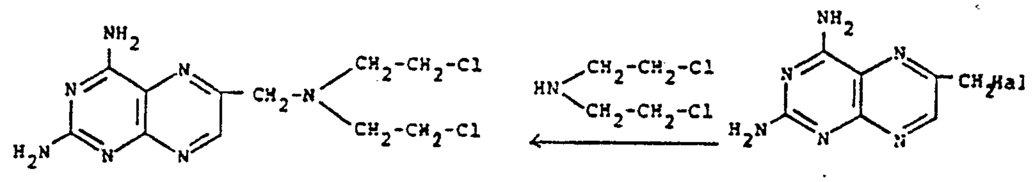

The 6-halomethyl derivative may be prepared by halogenation of hydrohalide salt of the aforesaid known intermediate in the presence of a catalyst and in an aprotic solvent. The reaction sequence would be as follows:

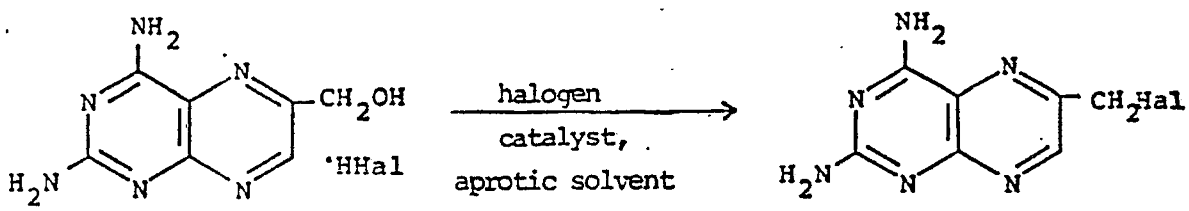


2,4-diamino-6-(bis-2-chloroethyl) aminomethyl pteridine

Appropriate catalysts in the above sequence would be, for example, triphenylphosphine and the like. Useful aprotic solvents include, among others, N,N-dimethylacetamide, N,N-diethylacetamide, 1-methylpyrrolidin-2 one, N,N-dimethylpropionamide, acetonitrile, benzonitrile tetrahydrofuran and dioxane.

The derivatives herein (which are inclusive of the pharmaceutically acceptable salts) can be formulated into a variety of dosage forms as is known in the art and utilized in the treatment of various cancers in the same manner as the currently used drug methotrexate.

The following examples illustrate methods for preparing the 2,4-diaminopteridine derivatives of this invention and their use in anticancer therapy.

EXAMPLE 1

Preparation of:

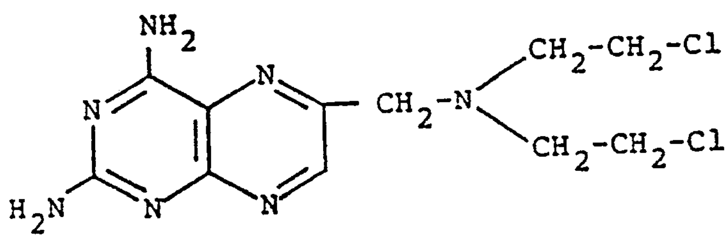

2,4-diamino-6-(bis-2-chloroethyl)aminomethyl pteridine

A. 2,4-Diamino-6-(hydroxymethyl)-pteridine hydrochloride

To a solution of 42.8 g (0.167 mol) of 2,4,5,6-tetraminopyrimidine sulfate monohydrate, which had been recrystallized from boiling 1 N sulfuric acid, in 700 ml of boiling water was added gradually a solution of 40.7 g (0.167 mol) of barium chloride dihydrage in 100 ml of water. After a short period of digestion, the mixture was filtered from the precipitated barium sulfate, the filtrate was stirred with 5 g of decolorizing carbon and filtered again. This solution was added to a stirred mixture of 426 g of ammonium chloride, 26.3 g (0.167 mol) of cysteine hydrochloride, 46.6 g (0.259 mol) of dihydroxyacetone dimer and 970 ml of water in a 3-1 three-necked flask equipped with a stirrer and a glass-fritted gas inlet tube. The resulting mixture was stirred for 67 hours at room temperature during which time a slow stream of oxygen was passed through the mixture via the gas inlet tube. The product, 2,4-diamino-6-(hydroxymethyl)-pteridine hydrochloride, separate as a tan solid which was collected by suction filtration, washed with water, and air dried; yield, 17.2 g (45%).

B. 2,4-Diamino-6-(bromomethyl)-pteridine hydrobromide

To a solution of 17.2 g of 2,4-diamino-6-(hydroxymethyl)-pteridine hydrochloride in 345 ml of hot water was added 50 ml of 28% aqueous ammonia. After cooling, the solid free base was filtered off and air dried; yield, 12.4 g (86%). A stirred mixture of this free base in 800 ml of boiling ethanol was treated with 7.5 ml of 48% hydrobromic acid and allowed to cool. The resulting hdyrobromide salt was collected by suction filtration, washed with ethanol and air dried; yield 16.6 g (94%).

To a stirred solution of 63.9 g (0.244 mol) of triphenylphosphine in 325 ml of N,N-dimethylacetamide was added dropwise 39 g (0.244 mol) of bromine at a temperature of 10 to 12° C. To this stirred mixture was added 16.6 g (0.061 mol) of 2,4-diamino-6-(hydroxymethyl)-pteridine hydrobromide in one portion. After stirring for 2 hours at 20-25° C, 4 ml of ethanol was added, and the mixture was kept in a cold room (4° C) overnight. Most of the N,N-dimethylacetamide was removed by distillation at a pressure of 1mmHg and a pot temperature not exceeding 45° C. The residue was stirred with 190 ml of benzene, which was decanted from the insoluble semisolid residue. This procedure was repeated with another 190 ml portion of benzene, which was decanted from the insoluble semisolid residue. This procedure was repeated with another 190 ml portion of benzene, and the solid that remained was stirred at 80° C with 435 ml of glacial acetic acid until solution was complete. This mixture was allowed to cool overnight, and the solid that separated was removed by filtration; yield 15.5 g. This crude product as recrystallized from boiling isopropanol, filtering from 5 g of insoluble material; yield of 2,4-diamino-6-(bromomethyl)-pteridine hydrobromide containing 0.5 mol of isopropanol or crystallation, 8.3 g (34.5).

C. 2,4-Diamino-6-bis(2-chloroethyl)aminomethyl pteridine

To a solution of 4 g of 2,4-diamino-6-(bromomethyl) pteridine hydrobromide in 150 ml of hot water was added 10 ml of 28% aqueous ammonia, and when cool the precipitated free base was filtered, washed with water, and air dried; yield, 2.94 g (94%).

To a stirred solution of 2.4 g (9.4 mmol) of 2,4-diamino-6-(bromomethyl)-pteridine in 50 nl of N,N-dimethylacetamide was added 6 g (0.04 mol) of bis(2-chloroethyl)amine, and the mixture was stirred at 50-55° C for 5 hours and then for 18 hours at room temperature. After filtering from a small amount of dark insoluble matter, the filtrate was diluted with 200 ml of water whereupon 2,4-diamino-6-bis (2-chloroethyl)-aminomethylpteridine separated as a light tan solid which was filtered, washed thoroughly with water and air dried; yield, 0.92 g (31%).

EXAMPLE 2

Preparation of:

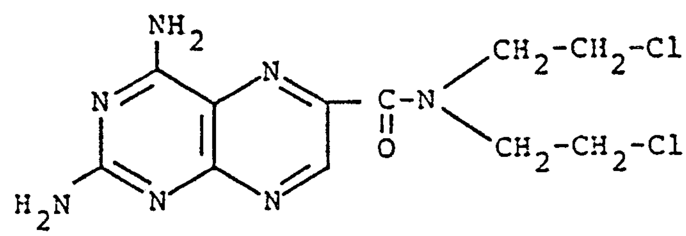

4 g of 2,4-diamino-6-(hydroxymethyl)-pteridine hydrochloride is dissolved in sodium bicarbonate and oxidized by $KNnO_4$. The oxidized product, 2,4-diamino-6(carboxy acid) pteridine, is obtained by neutralizing the reaction mixture with diluted $H_2SO_4$.

Synthetic Route 1: 2.5 g (9.5 mmol) of 2,4-diamino-6(carboxy acid)pteridine is dissolved in DMSO and added to 9.5 mmol of N,N'-dicyclohexylcarbodiimide (DCCI). To this reaction mixture is added 6 g (0.4 mmol) of bis(2-chloroethyl) amine and stirred for 18 hours at room temperature. The reaction mixture is then worked up in the same way as mentioned in step C, Example 1.

Synthetic Route 2: 2.5 g (9.5 mmol) of 2,4-diamino-6(carboxy acid)pteridine is dissolved in dry DMF. The solution is cooled to -5° C. To this solution was added 9.5 mmol of N-methylmorpholine and stirred for 15 minutes. To the stirred solution was added 9.5 mmol of ethylchloroformate and stirred for 1/2 hour. A

freshly prepared bis(2-chloroethyl) amine (0.5 mmol) in DMF (chilled) was added dropwise. After 1/2 hour the reaction mixture is brought to room temperature and is left overnight. The reaction mixture is evaporated to dryness. The residue is dissolved in water and neutralized by adding diluted HCl or $NaHCO_3$ solution. The rest of the procedure is same as stated above.

EXAMPLE 3

The potency of the compound of Example 1, i.e., 2,4-diamino-6(bis-2-chloroethyl)aminomethyl pteridine, was evaluted in B6D2F1/J female mice, (Jackson Lab., Bar Harbor, ME). During the course of the experiments, the animals were maintained in a controlled environment with limited accessibility and were permitted free access to food and water. They were divided in groups containing 10 animals in each group. The mice were inoculated intraperitoneally (I.P.) with $1X10^5$ L-1210 mouse leukemia lymphoblasts. Day zero was the day when L-1210 was injected and the following day, Day 1, the treatment started. Initially the doses of the drug used were 2.5, 5, 10 and 20 mg/kg and they were used as suspension in distilled water. For the purpose of studying the apparent toxicity of this drug the above doses were also injected (I.P.) in normal mice of 9-11 weeks of age weighing 22-25 g per mouse. A volume of 0.2 to 0.3 ml of the suspension was injected in each animal. A single dose was used for both toxicity and activity studies.

The results from the toxicity studies showed no toxic death of animals from the dose that showed maximum activity, i.e., 5 mg/kg. The results of the drug activity is summarized in Figure 1. As it can be seen, the drug had significant effect on the survivability of animals implanted with $1X10^5$ L-1210 tumor cells on Day 0. Several doses were examined and in each instance there was noticeable prolongation of lifespan compared to the control (broken line in Figure 1). For example, animals receiving 2.5 mg/kg of the drug had an increased lifespan of 190%, while animals receiving 5 mg/kg dose showed a remarkable survivability (solid line in Figure 1). This dose was repeated twice and in each study it showed the similar survival pattern, i.e., 50% of the animals had an increased lifespan of more than 200%, while the other 50% of the animals were totally cured and even remained alive at the end of 90 days. However, those animals receiving the drug at 10 and 20 mg/kg extended the lifespan 250 and 140%, respectively, and 20% of the animals treated with 10 mg/kg remained alive at the end of 30 days (see Figure 1).

The activity of 2,4-diamino-6-(bis-2-chloroethyl) aminoethyl pteridine was also tested on solid tumor. BALB/C adult female mice were given $2X10^6$ viable MOPC 104E cells intravenously. 18 Days following the transplantation of tumor, when the tumor burden was large and at an advanced stage, the mice were treated with 5 mg/kg or our compound in suspension (I.P.). The increase in lifespan of mice was found to be 224% even when the treatment started at a very advanced stage of tumor growth (i.e., treatment started on the 18th day while the control died on the 20th day).

## Claims

**1.** Pteridine compounds of the formula:

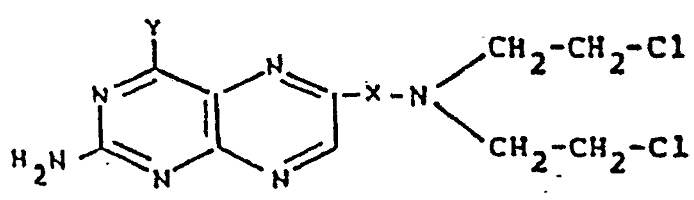

wherein
Y is $NH_2$, OH or SH, and
X is

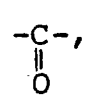

or $-CH_2-$.

**2.** A compound of Claim 1 of the formula:

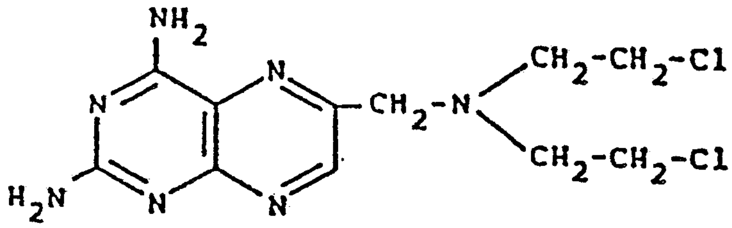

3. An anticancer composition containing an anticancer-effective amount of a compound of Claim 1.

4. An anticancer composition containing an anticancer-effective amount of a compound of Claim 2.

5. A process of preparing compounds of the formula:

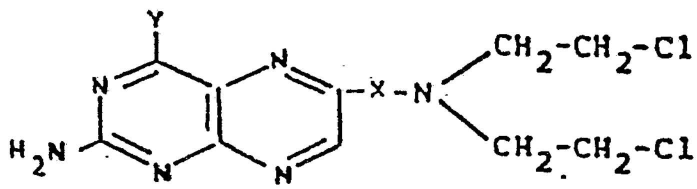

wherein
Y is $NH_2$, OH or SH, and
X is

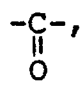

or $-CH_2-$
which comprises
when X is $CH_2$, reacting a methyl halide of the formula

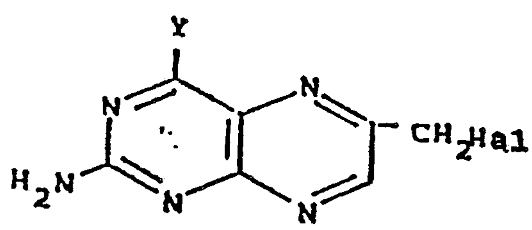

with an amine of the formula

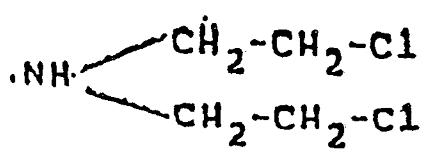

or when X is

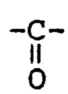

reacting a carboxylic acid of the formula

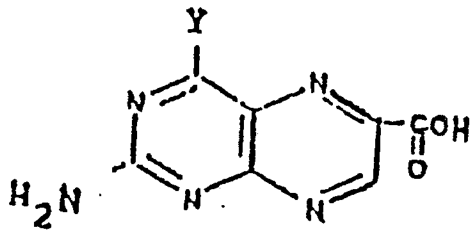

under amide forming conditions, with an amine of the formula

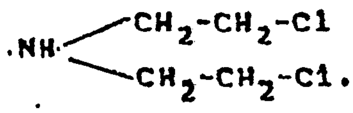

6. The process according to Claim 5 for preparing the compound of the formula:

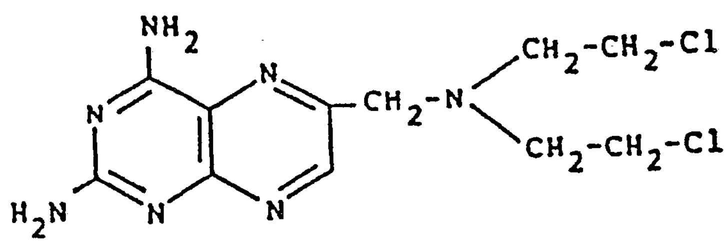

**Revendications**

1. Composés de ptéridine représentés par la formule :

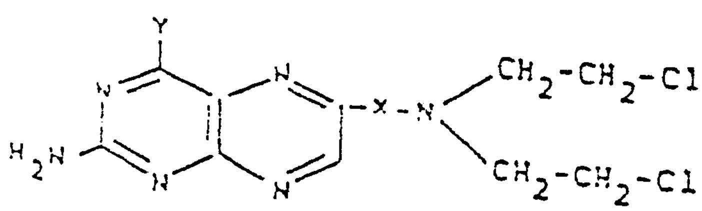

dans laquelle :

- Y représente $NH_2$, OH ou SH ; et
- X représente

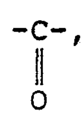

ou $-CH_2-$.

2. Composés selon la revendication 1, représentés par la formule :

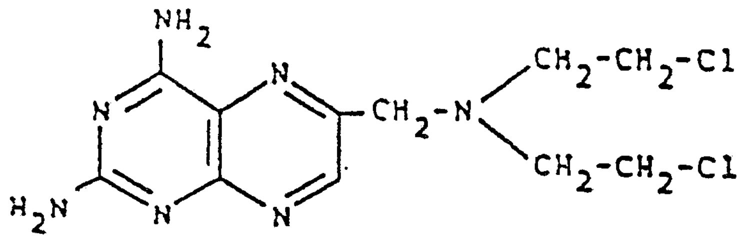

3. Composition anticancéreuse contenant une quantité ayant une efficacité anticancéreuse d'un composé de la revendication 1.

4. Composition anticancéreuse contenant une quantité ayant une efficacité anticancéreuse d'un composé de la revendication 2.

5. Procédé de fabrication de composés représentés par la formule :

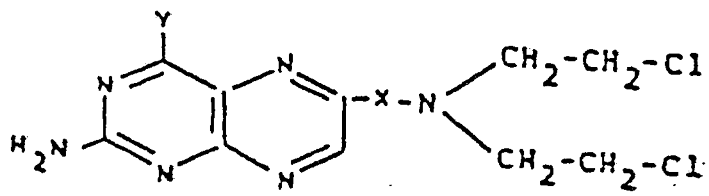

dans laquelle :

- Y représente $NH_2$, OH ou SH ; et
- X représente

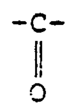

ou $-CH_2-$

qui comprend :

lorsque X représente $CH_2$, la réaction d'un halogénure de méthyle représenté par la formule :

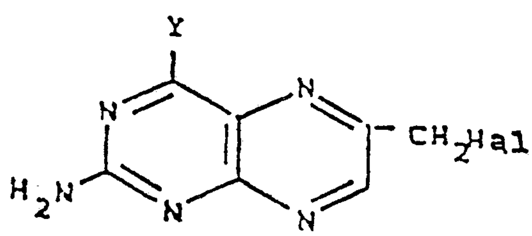

avec une amine représentée par la formule :

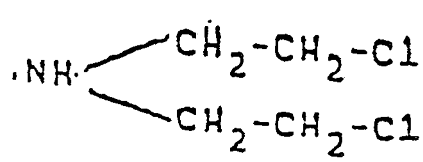

ou, lorsque X représente

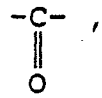

la réaction d'un acide carboxylique représenté par la formule:

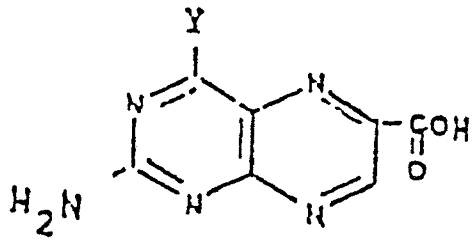

dans des conditions de formation d'amide, avec une amine représentée par la formule :

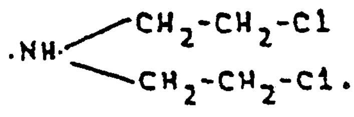

6. Procédé selon la revendication 5, pour fabriquer le composé représenté par la formule :

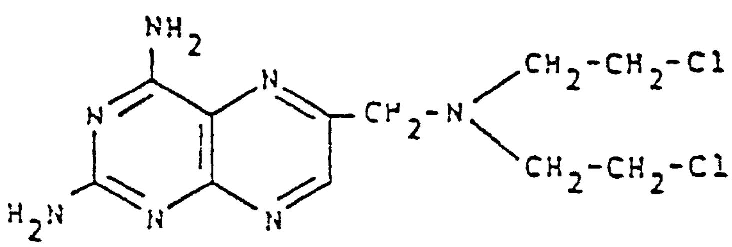

**Patentansprüche**

1. Pteridinverbindungen der Formel

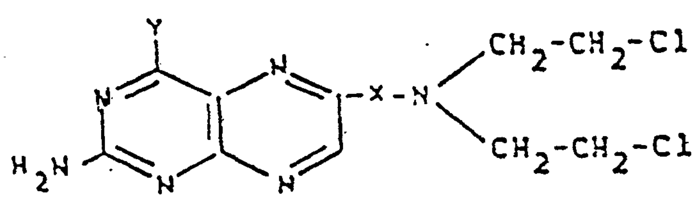

worin
Y $NH_2$, OH oder SH ist und
X

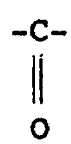

oder $-CH_2-$ ist.

2. Verbindung gemäss Anspruch 1 der Formel

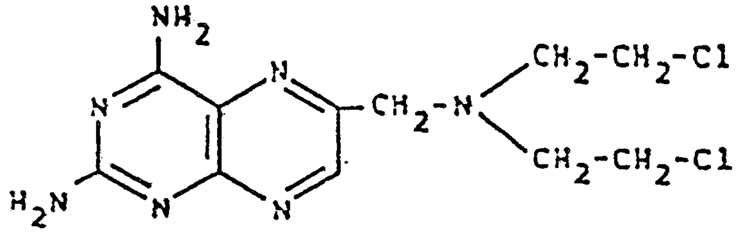

3. Antikrebszusammensetzung, enthaltend eine gegen Krebs wirksame Menge der Verbindung von Anspruch 1.

4. Antikrebszusammensetzung, enthaltend eine gegen Krebs wirksame Menge einer Verbindung gemäss Anspruch 2.

5. Verfahren zur Herstellung von Verbindungen der Formel

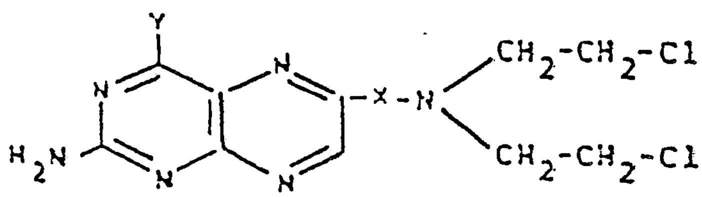

worin
Y $NH_2$, OH oder SH ist und
X

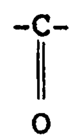

oder $-CH_2-$ ist.
umfassend, für den Fall, dass X $-CH_2-$ ist, das Umsetzen eines Methylhalogenids der Formel

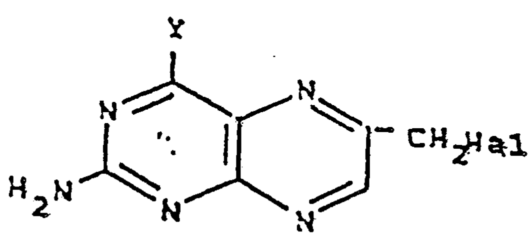

mit einem Amin der Formel

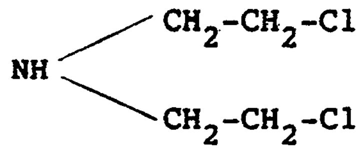

oder, wenn X

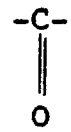

ist, das Umsetzen einer Carbonsäure der Formel

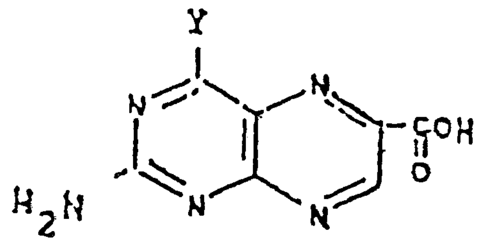

unter amidbildenden Bedingungen mit einem Amin der Formel

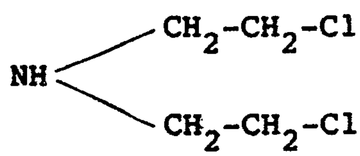

**6.** Verfahren gemäss Anspruch 5 zur Herstellung der Verbindung der Formel

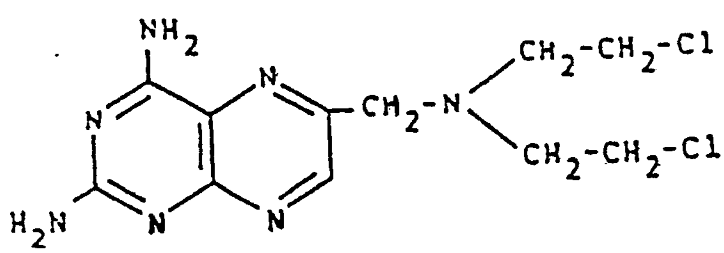

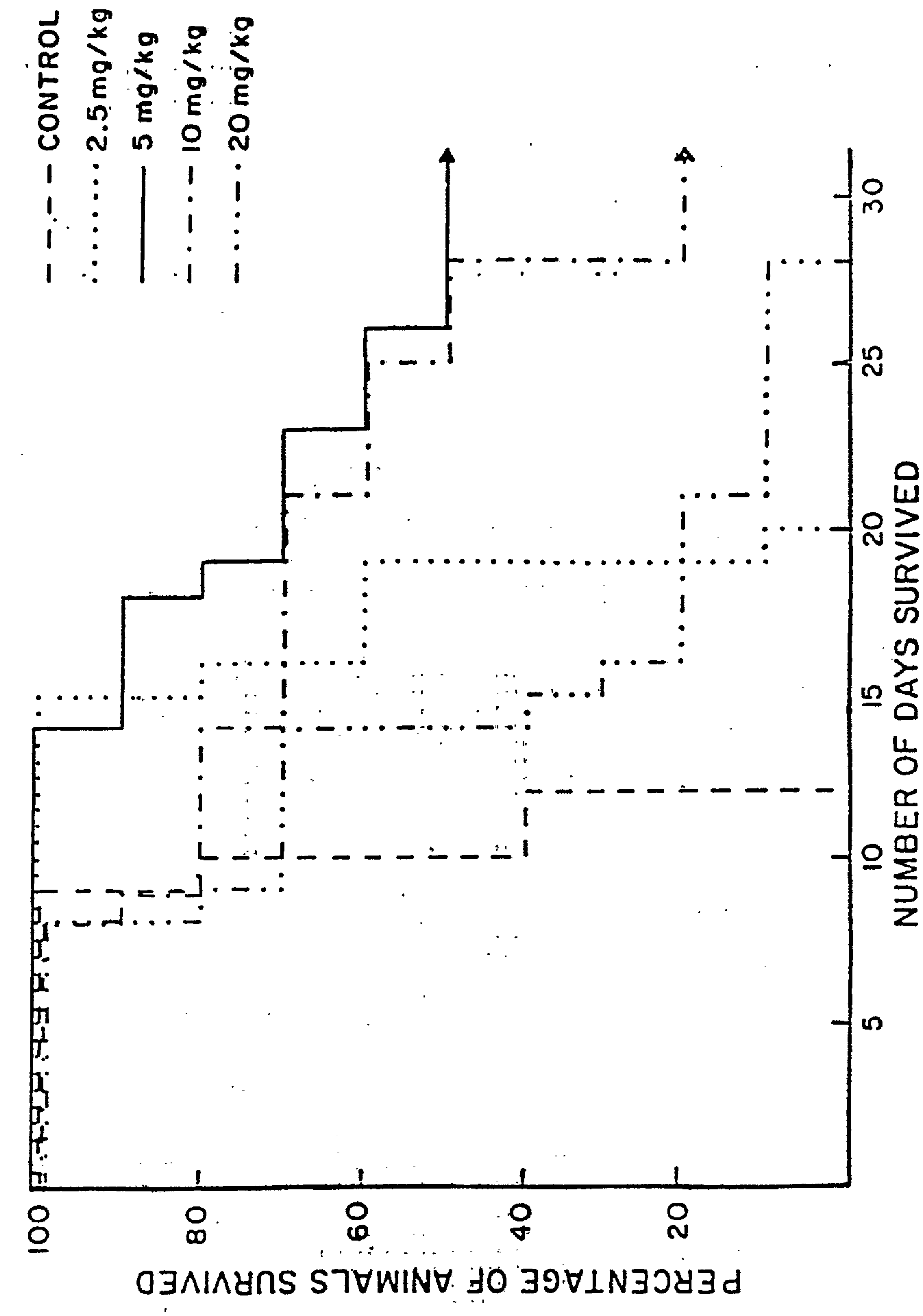
CONTROL
2.5 mg/kg
5 mg/kg
10 mg/kg
20 mg/kg
100
80
60
40
20
PERCENTAGE OF ANIMALS SURVIVED
5
10
15
20
25
30
NUMBER OF DAYS SURVIVED